# EUROPEAN PATENT SPECIFICATION

(11) **EP 2 641 878 B1**
(45) Date of publication and mention of the grant of the patent: **22.10.2014**
(21) Application number: 12382105.0
(22) Date of filing: 22.03.2012
(51) Int. Cl.: C02F 9/00, C07D 301/19, C07D 301/32, C22B 7/00, C22B 3/42, C22B 34/32, B01J 38/00, B01J 41/04, B01J 47/02, B01J 49/00, C02F 103/36, C02F 1/42, C02F 1/66, C02F 1/72, C02F 11/08

(54) **Molybdenum and benzoic acid recovery from aqueous waste streams generated in epoxidation processes**
Rückgewinnung von Molybdän- und Benzoesäure aus wässrigen Abfallströmen, die bei Epoxidationsverfahren erzeugt werden
Récupération d'acide benzoïque et de molybdène à partir de flux de déchets aqueux générés lors de procédés d'époxydation

(43) Date of publication of application: 25.09.2013
(73) Proprietor: Repsol, S.A., 28045 Madrid (ES)
(72) Inventor: Rodríguez Bustamante, Jorge, 28935 Móstoles (ES); Masa Lorenzo, María, 28935 Móstoles (ES); García Biosca, Eva María, 28935 Móstoles (ES); De Frutos Escrig, Pilar, 28935 Móstoles (ES); Mula Andrés, Belén, 28935 Móstoles (ES); Bustos Franco, César, 28935 Móstoles (ES)
(74) Representative: ZBM Patents - Zea, Barlocci & Markvardsen

(56) References cited:
- US-A- 3 988 353
- US-A- 5 171 868
- US-A1- 2004 138 482
- US-A1- 2004 217 062

## Description

The present invention relates to a method for the revaluation of aqueous waste streams generated in an epoxidation processes. In particular, it discloses a process of recuperation of molybdenum and benzoic acid values in such epoxidation process.

### BACKGROUND ART

The production of oxirane compounds such as propylene oxide by the catalytic reaction of an olefin with an organic hydroperoxide is a process of great commercial importance. Molybdenum containing catalysts are very suitable for carrying out the reaction under homogeneous conditions due to its high reactivity and therefore the possibility of working with very small concentrations.

One of the most relevant epoxidation processes of this technology is the co-production of propylene oxide and styrene monomer (PO/SM).

The process for the co-production of propylene oxide and styrene involves the molecular oxygen oxidation of ethylbenzene to form ethylbenzene hydroperoxide; the catalytic reaction of the ethylbenzene hydroperoxide with propylene to form propylene oxide and methylbenzyl alcohol; and the dehydration of the methylbenzyl alcohol for the production of styrene monomer.

Different acid and phenolic compounds are formed in the two first stages. The presence of these acid and phenolic compounds, together with the catalyst used in the epoxidation is harmful downstream of the process, since they could give rise to problems in columns and reactors after epoxidation. Therefore, it is totally essential to perform and alkaline washing of the epoxidation outlet stream in order to eliminate them.

This alkaline washing produces an aqueous stream, also called alkaline purge, containing molybdenum, sodium and highly loaded in organic compounds which can be taken integrally to water treatment, without any prior pre-treatment, which permits the recovery of organic matter and molybdenum. The alkaline purge comprises generally between 4-8 % of compounds of alcoholic nature, largely monopropylene glycol and methylbenzyl alcohol; 3-6 % of organic salts, largely sodium benzoate and phenolate; and a content greater than 2 % of sodium hydroxide.

In the patent application EP 2277834 is described a process to treat the alkaline purge with advantages in relation with incineration or oxidation. The treatment comprises the following steps:
a) acidification of the aqueous stream with an inorganic acid to separate two phases, one aqueous and another organic;
b) separation of the phases,
c) washing of the organic phase with an aqueous solution of acid
d) separation of the two phases produced in c).

On the other hand, through these procedure is possible the recovery of the molybdenum from the aqueous phase separated by the use of ion exchange resins, which is not possible in an alkaline stream rich in organic compounds, but it is feasible in an acid stream with lower content in said compounds. In this process the organic phase recuperated is a mixture of organic compounds and it is complicated to recuperate no one, the organic phase is used as fuel.

In the patent US 558507 is described a method to recover molybdenum, wherein the aqueous process stream containing molybdenum and sodium values as well as organics is treated, as by incineration, to first separate organics. During the incineration process, particulate ash comprised of the molybdenum and sodium values, passes downwardly through the incinerator with the incinerator gases. The ash-containing gases are quenched by admixing with water to form the incinerator blowdown. The blowdown is an aqueous solution of the molybdenum value, as sodium molybdate, and sodium value, as sodium carbonates, from the epoxidation process stream. The aqueous blowdown stream is acidified, as with HCl, so that carbonates are converted to CO₂ which can readily be removed. Thereafter, the essentially carbonate-free stream is treated at carefully controlled conditions to convert molybdenum to CaMoO₄ which is precipitated from solution and recovered. In this patent is indicated that, in place of incineration, other procedures such as wet air oxidation or biotreatment may be used to remove organic materials before conversion of molybdenum values to CaMoO₄ but incineration does, however, represent the preferred method for eliminating the organic materials.

In the patent EP 0918763 is described another alternative method to recover the molybdenum from the alkaline purge. The aqueous epoxidation process stream containing molybdenum and sodium values as well as organics is treated by known methods, as by incineration, to first separate organics prior to the carbon bed treatment. The substantially organics free aqueous stream, is acidified, as with H₂SO₄, so that carbonates are converted to CO₂ which can readily be removed. Thereafter, the essentially carbonate-free stream is contacted with solid activated carbon adsorbent to separate molybdenum values contained therein. The resulting aqueous solution greatly reduced in contained molybdenum can then be conveniently disposed of, if maximum molybdenum specifications are very stringent, the aqueous effluent from the carbon treatment is further treated by contact with a basic ion exchange thereby to effectuate substantially complete molybdenum removal. According with this patent in place of incineration, other known procedures such as wet air oxidation or biotreatment may be used to remove organic materials prior to carbon treatment but incineration does, however, represent the preferred method for eliminating the organic materials.

In the article "Conceptual process design as a prerequisite for solving environmental problems: a case study of molybdenum removal and recovery from wastewater" Mineral Engineering . 2004, vol. 17, p.205-215 the specific case involving a new styrene monomer plant is analyzed. The process option that produces CaMoO₄ by precipitation was selected as the most attractive molybdenum recovery process after evaluation of the designs against relevant quality factors like safety, economics, product market, environmental regulations and chemistry. For this option it is necessary to incinerate the alkaline purge and acidification before the treatment to eliminate the organic compounds.

All of these previous publications describe process in which it is performed the combustion of organic compounds by incineration or using these organic compounds as fuel. It is impossible to recuperate any organic compound in addition with molybdenum.

The wastewater treatment of the PO/SM and derivatives wastewater with solvent extraction provides unsatisfactory results, since those treatments showed high losses of solvent, insufficient extraction, and a final effluent with a high solid content, a high coloration, and a high chemical oxygen demand (COD) value. On the other hand, incineration treatments require high energy consumption, whereas the high salt content of the wastewater further complicate incineration operation.

On the other hand, benzoic acid is usually prepared by a process such as oxidation of toluene. Benzoic acid obtained by a process such as mentioned above contains a small amount of impurities even after removal of low-boiling substances such as toluene beforehand. The impurities here include benzoic acid esters, biphenyls, p-phenylbenzoic acid, phthalic anhydride and toluic acid and, as they cause problems relating to emission of odour and coloration, they should desirably be removed from the product benzoic acid.

Several processes are known for the purification of benzoic acid, among others vaporization in an inert gas followed by crystallization under specified conditions as disclosed in US 4227018; distillation in the presence of aliphatic amines as disclosed US 4092353; and purification by supercritical extraction as disclosed in US 4652675; process based on crystallization such as recrystallization as disclosed in US 6211404. None of these processes, however, satisfied all the factors involved in purification such as efficiency, equipment, and cost.

Accordingly, there exists the necessity of a suitable method for the revaluation of aqueous waste streams generated in the epoxidation processes, avoiding the expensive and polluting processes of incineration, wet air oxidation at high temperatures and pressure or a combination thereof, and which allows a reduction of the environmental impact, reducing the organic load of the waste flows and optimising subsequent treatments.

Molybdenum from aqueous concentrates can be recovered by different unit operations, i.e. evaporation or chemical precipitation. Chemical precipitation is preferred when the concentration and purity of molybdenum concentrates is lower. Molybdenum can be recovered by chemical precipitation with calcium salts to form insoluble calcium molybdate, hydrogen sulphide or sodium hydrosulphide to form insoluble molybdenum sulphide or iron sulphate to form a mixed iron and molybdenum oxide.

The patent US 3988353 discloses the recovery of a molybdenum catalyst from an epoxidation effluent by contacting the effluent with an aqueous basic solution and subsequent removal of molybdenum from the organic phase.

In the patent CA1116408 A1 is described a process for the recovery of molybdenum values from mining wastewater by chemical precipitation followed by gas flotation. Molybdenum values are recovered by addition of a mineral acid to wastewater to lower its pH and a trivalent metal to the water to form an insoluble precipitate. The pH is raised with a strong base to improve the recovery and the solid precipitate separated from the wastewater by gas flotation. Another examples of similar practices are found in the North Metal & Chemical Company Technical Bulletin: Molybdenum removal procedure (www.nmc-nic.com), and in Sutulov A., 1979, International Molybdenum Encyclopaedia, Vol. II: Processing and Metallurgy, Internet Publications, pp. 250-252.

### SUMMARY OF THE INVENTION

The present invention relates to a method for the revaluation of aqueous waste streams generated in epoxidation processes, preferably in the propylene oxide and styrene co-production processes (PO/SM). In particular, it relates to a process of recuperation of molybdenum and benzoic acid from the alkaline purge resulting of the alkaline washing of the epoxidation outlet stream in the epoxidation process.

In accordance with the present invention, the alkaline purge stream containing molybdenum and sodium values as well as organics is treated by wet air oxidation (WAO) to generate a stream comprising sodium molybdate, sodium benzoate, sodium carbonate and sodium acetate as majority components. The resulting stream, WAO effluent, may not be directly discharged to the environment due to the presence of the molybdenum and organic matter contained therein. Thus, in accordance with the present invention, the WAO effluent resulting from the alkaline purge wet air oxidation is acidified, preferably with an inorganic acid at a pH comprised of from 1.0 to 4.5, to eliminate the carbonate as carbon dioxide and produce the benzoic acid precipitation.

Accordingly, a solid-phase fraction containing impure benzoic acid and a liquid-phase fraction containing molybdenum are obtained in the acidification step. Thereafter, the solid-phase fraction and the liquid-phase fraction are separated, preferably by filtration, to obtain a solid-phase containing impure benzoic acid and a liquid-phase containing molybdenum. The solid-phase is washed and dried to obtain benzoic acid. Whereas the liquid-phase stream is passed through at least one anionic exchange resin to produce a liquid stream with low molybdenum concentration. The regeneration of the anionic exchange bed with a base allows recuperating the molybdenum as molybdate. The resulting aqueous stream with a reduced content in molybdenum, preferably of less than 5 ppm, more preferably less than 2 ppm can be conveniently disposed of after further conventional treatment, e.g. biological treatment in the presence or absence of activated carbon.

### BRIEF DESCRIPTION OF THE DRAWINGS

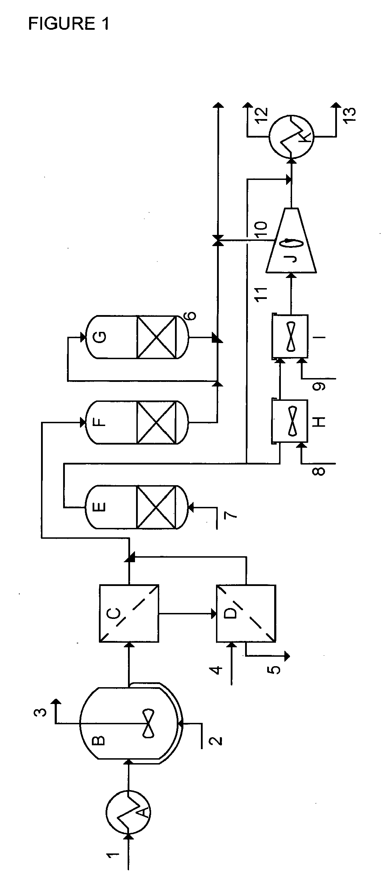

Figure 1 shows a simplified process scheme. A wet air oxidation effluent (1) is cooled in a heat exchanger device (A). A mineral acid (2) is added to the cooled effluent in a stirred tank (B). As a result of acidification carbon dioxide (3), a solid fraction rich in benzoic acid and a liquid fraction retaining the molybdenum values are produced. The benzoic acid rich solid fraction is separated from the effluent in a physical separation device, i.e. a press filter (C).

The solid fraction obtained in (C) is purified into another physical separation device (D). Demineralised water (4) is added to purify the benzoic acid in a physical separation device, i.e. a nutsche filter, via resuspension and filtration or cake washing, and subsequent drying with air or nitrogen at a temperature. A purified benzoic acid material is obtained (5). Washing water is mixed with the liquid fraction retaining the molybdenum values.

The liquid fraction retaining the molybdenum values obtained in (C) is subjected to an ion exchange carrousel (E, F, G). After passing through the beds in service mode (F, G) a liquid with reduced values of molybdenum is obtained (6). The molybdenum is accumulated in the ion exchange beds and extracted during regeneration mode (E) with a strong base (7) to obtain a molybdenum concentrate.

The molybdenum concentrate from the ion exchange beds is chemically precipitated in a stirred tank (H) by addition of a mineral acid and an iron sulphate solution (8). Molybdenum recovery is improved in another stirred tank (I) by addition of a strong base (9) and a molybdenum rich solid suspension is obtained. The molybdenum rich solid suspension is concentrated by means of centrifugal separation devices (J), and as a result a liquid phase with reduced molybdenum content is obtained (10). The suspension is dried by a heat exchanging device (K), water is eliminated (12) and a dry molybdenum rich solid is obtained (13).

Alternatively, the molybdenum concentrate from the ion exchange beds is evaporated by a heat exchange device (11).

### DETAILED DESCRIPTION OF THE INVENTION

According to an embodiment of the invention, the carbon dioxide generated can be recuperated in form of sodium carbonate to be used in the propylene oxide and styrene co-production process.

In another embodiment of the invention, the carbon dioxide generated can be recuperated by means of absorption to be used as an acid in different processes, e.g. biological treatment.

In another embodiment of the invention, the molybdenum values recuperated as molybdate can be transformed in epoxidation catalyst precursor and be used as catalyst in the epoxidation reaction.

The first step of the process according to the present invention is the wet air oxidation (WAO) of the alkaline purge resulting of the high strength PO/SM wastewater. The wet air oxidation process is an oxidation reaction that occurs in liquid water. The reactions occur at much lower temperatures than incineration, and elevated pressure is required to maintain water in the liquid phase. Because the liquid is not vaporized, wet air oxidation requires less energy for autothermal operation than incineration. The hydrocarbons are converted to CO₂ and water. Oxidation is rarely complete so a portion of the organic COD remains as readily biologically treatable organic acids, such as acetic acid.

According to an embodiment of the present invention, the wet oxidized effluent exhibits a 5-days biological oxygen demand (BOD₅): chemical oxygen demand (COD) ratio comprised of from 0.6 to 0.8, preferably 0.7. Thus, the WAO effluent is readily biodegraded with high efficiency and toxicity problems in subsequent biological process are overcome.

According to an embodiment, the operating conditions for the wet air oxidation step are a temperature comprised of from 250 to 315°C, a pressure comprised of from 40 to 120 bar, a COD inlet concentration comprised of from 5 to 17% wt, a reaction time comprised of from 20 to 120 min, and excess of compressed oxygen, air, or their mixture as the oxidant, in order to obtain an oxygen concentration comprised of from 1 to 15% vol in the WAO off-gas.

The preferred operating conditions for the wet air oxidation are a temperature comprised of from 255 to 295°C, a pressure comprised of from 50 to 95 bar, a COD inlet concentration comprised of from 12 to 16% wt, a reaction time comprised of from 30 to 100min, and excess of a compressed mixture of oxygen and air, with an oxygen content comprised of from 50 to 90% vol, in order to obtain an oxygen concentration comprised of from 2 to 10% vol. in the WAO off-gas.

Most preferred parameters for the wet air oxidation are an inlet reactor temperature comprised of from 225 to 280 °C, an outlet reactor temperature comprised of from 285 to 295°C, a pressure comprised of from 93 to 95 bar, a COD inlet concentration comprised of from 13 to 15% wt, a reaction time comprised of from 60 to 90 min, and excess of a compressed mixture of oxygen and air, with an oxygen content comprised of from 75 to 85% vol, in order to obtain an oxygen concentration comprised of from 4 to 8% in the WAO off-gas.

In accordance with another embodiment, the WAO effluent is characterized by a composition comprised of from 1.6 to 2.7 % sodium acetate, 0.7 to 1.1 % sodium benzoate, 0.5 to 0.8 % sodium carbonate, 0.06 to 0.10% sodium propionate, 0.06 to 0.10% sodium formate and a chemical oxygen demand content comprised of from 2.9 to 4.9%.

Reduction of the precipitation temperature of benzoic acid results in an increase in the precipitation yield.

According to an embodiment of the present invention, previously to the acidification step the wet air oxidation effluent is cooled down at a temperature range comprised of from 2 to 50 °C, preferably comprised of from 5 to 40 °C, more preferably at a temperature comprised of from 6 to 15 °C. Thus, the acidification step is carried out at such temperature. In a particularly preferred embodiment the acidification step is carried out at a temperature of 10 °C.

Acidification of the wet oxidized effluent (WAO effluent) results in eliminating the carbonate as carbon dioxide and producing the benzoic acid precipitation.

The acidification step is carried out by addition of an inorganic acid, preferably selected from sulphuric acid, hydrochloric acid, nitric acid and phosphoric acid. Preferably the acid is sulphuric acid.

According to an embodiment, the pH of the stream is downed until pH comprised of from 1.0 to 4.5, preferably pH comprised of from 1.5 to 3.0. It is particularly preferred to down the pH comprised of from 2.0 to 2.5.

In a particularly preferred embodiment, the wet air oxidation effluent of step (i) is cooled down at a temperature comprised of from 6 to 15°C and then the stream is acidified at a pH comprised of from 2.0 to 2.5.

As a consequence of the acidification, a solid-phase fraction containing impure benzoic acid and a liquid-phase fraction containing molybdenum are obtained. Several techniques generally known by the skill person can be used in order to separate the solid from the aqueous phase. The use of a polymeric membrane with an average pore size ranging from 0.15 to 100.00 µm is preferred. Being more preferred the use of a polymeric membrane with an average pore size ranging from 1.00 to 50.00 µm. Particularly preferred are those polymeric membranes with an average pore size ranging from 10.00 to 40.00 µm.

In accordance with a particular embodiment, the feeding is recirculated several times through the filter.

According to an embodiment, the benzoic acid can be separated at filtration speeds comprised of from 3 to 100 m³/h/m² at 1 bar pressure, preferably comprised of from 6 to 50 m³/h/m² at 1 bar pressure.

The wet cake thus obtained is washed in order to purify the benzoic acid. Preferably, the washing step is performed with demineralised water, preferably at a temperature comprised of from 10°C to 60°C, more preferably comprised of from 20°C to 30°C, resulting in the recovery of benzoic acid.

According to another embodiment, the washing step is performed by suspension of the wet cake and careful stirring, preferably during 5-20 min, more preferably during 10 min. In such a case, it is necessary a water:dried cake ratio comprised of from 50:1 to 2:1 w/w, preferably 10:1 to 5:1 w/w, being particularly preferred 8.5:1 w/w.

Alternatively, the washing step is performed by permeation of fresh water through the cake, being necessary a water : dried cake ratio comprised of from 50:1 to 10:1 w/w, preferably comprised of from 20:1 to 15:1 w/w, being particularly preferred 16:1 w/w.

According to an embodiment of the present invention, the process further comprises drying the washed solid phase obtained in step (iv), preferably in presence of an air or nitrogen stream, at a temperature comprised of from 5 to 100°C, preferably comprised of from 40 to 80°C, more preferably from 50 to 75°C, being particularly preferred at 60°C, and recovering the benzoic acid.

Thus, the benzoic acid can be obtained with a purity of at least 95 %, preferably at least 98 %; with a sodium content lower than 3000 ppm, preferably lower than 1000 ppm; and a molybdenum content lower than 100 ppm, preferably lower than 50 ppm.

The liquid-phase containing molybdate obtained in the separation step, after the acidification of the WAO effluent, is brought into contact with an anionic exchange resin to adsorb molybdic acid ions, by passing it through an anionic exchange resin, producing a liquid stream with low molybdenum concentration. In an embodiment of the present invention, the anionic exchange resin is a weak anionic resin, preferably a macroporous polystyrene crosslinked with divinylbenzene supported anionic resin, being particularly preferred those resins having ternary, quaternary or complex amines as functional group. In accordance with an embodiment of the present invention, the volumetric capacity of the resins is comprised of from 1.2 to 1.9 eq/L.

According to an embodiment, the aqueous phase containing molybdate is adjusted to pH comprised of from 1.0 to 4.5, preferably pH comprised of from 1.5 to 3.0, being particularly preferred comprised of from 2.0 to 2.5; and the ion exchange step is performed at a temperature comprised of from 10 to 40 °C, preferably comprised of from 15 to 30 °C, being particularly preferably at room temperature.

In accordance with an embodiment, the operating conditions of the resin are:
- a service flow rate comprised of from 1 to 20 BV/h, preferably comprised of from 3 to 10 BV/h, being particularly preferably at a flow rate of 5 BV/h;
- a bed depth comprised of from 0.25 to 2.50 m, preferably comprised of from 0.75 to 2.00 m;
- using cycles of 3-24 h, preferably 4-12 h, being particularly preferred 8 h.

Thereafter, the anionic exchange resin having molybdate ions is brought into contact with an alkali aqueous solution with pH of at least 8 to regenerate the anionic exchange resin, allowing dissolving molybdate ions to recover them as molybdate and a waste aqueous stream with a content in molybdenum of less than 5 ppm, preferably less than 2 ppm, which can be treated with a known method in order to be disposed of to the environment.

According to an embodiment of the present invention, the regeneration of the anionic exchange resin is performed with an alkali aqueous solution of a base selected from NaOH, NH₄OH, Na₂CO₃ preferably NaOH at a concentration comprised of from 2 to 8 %, or NH₄OH at a concentration comprised of from 2 to 10 %. It is particularly preferred NaOH at a concentration of 4%. The regeneration of the resin is carried out with passing the alkali aqueous solution through the resin preferably at a flow rate comprised of from 1 to 14 BV/h, preferably 2-8 BV/h, being particularly preferred a flow rate of 4 BV/h, operating in counterflow.

The waste aqueous stream obtained after passing one bed contains less than 50 ppm of molybdenum, preferably less than 30 ppm, and more preferably less than 20 ppm.

In accordance with an embodiment, the aqueous phase containing molybdate obtained in the separation step is treated in a carousel of at least three anionic exchange resin beds, passing through two beds in series while the third bed is in regeneration. Thus, according to the present invention, it is possible to obtain a final waste aqueous stream, after passing two beds in series, with molybdenum content of less than 5 ppm, preferably less than 2 ppm.

Molybdenum values can be recovered from the molybdate solution by a known method. According to an embodiment of the invention, molybdenum values are recovered from the molybdate solution by chemical precipitation with a commonly used reactant, preferably the reactant is selected from CaCl₂, CaSO₄, Ca(OH)₂, Fe(III) citrate, FeCl₃, Fe₂(SO₄)₃, FeSO₄. Particularly preferred is the use of FeSO₄, Fe₂(SO₄)₃ and CaCl₂; being more preferably Fe₂(SO₄)₃. The molybdenum is thus precipitated in the form of an insoluble salt or oxide.

When Fe₂(SO₄)₃ is used, in order to precipitate the molybdenum, it is preferably to use a Fe:Mo ratio (molar) comprised of from 0.4:1 to 50:1, more preferably comprised of from 6:1 to 15:1, being particularly preferred a Fe:Mo ratio (molar) of 8:1. Preferably, the pH is maintained comprised of from 2 to 5, more preferably comprised of from 3 to 4, particularly preferred pH comprised of from 3.2 to3.5.

The temperature of precipitation is preferably comprised of from 25 to 100 °C, more preferably comprised of from 60 to 90 °C, being particularly preferred 80 °C.

In the above embodiment, when Fe₂(SO₄)₃ is used to precipitate the molybdenum, the effluent containing the insoluble molybdenum salt or oxide is contacted with an alkaline stream to rise the pH comprised of from 3.7 to 8, most preferably to have a pH of 7.

The basification step is carried out by addition of a strong base. Sodium hydroxide, potassium hydroxide or calcium hydroxides are preferred. A 20 % Ca(OH)₂ whitewash is particularly preferred;

The molybdate containing slurry thus obtained is physically concentrated and dried to recover a molybdenum concentrate with a solid content of at least 80 % and a molybdenum content comprised of from 7 to 10 % on dry basis.

According to an embodiment of the present invention, the process further comprises the dissolution in H₂O or in aqueous NaOH of the CO₂ formed during the acidification step (ii).

As used herein, the following terms are used to describe the present invention. The definitions provided below, within context, may be used exclusively, or may be used to supplement definitions which are generally known to those of ordinary skill in the art.

### DEFINITIONS

lon exchange carrousel: is a typical ion exchange configuration formed by three or more ion exchange beds which periodically shift operating mode: In the three bed configuration, the liquid fraction passes through two beds in service mode: first and second stage beds, while the third bed is in regeneration, washing and conditioning mode prior to become in service mode. When the outlet concentration of the first stage bed reaches a certain molybdenum value, the beds are switched as described: The bed servicing in first stage mode is regenerated, washed, and conditioned; the bed servicing in second stage mode starts servicing in first stage mode; the conditioned bed starts servicing in second stage mode.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in the examples, are solely for attempting to increase the intelligibility of the claim. Furthermore, the present invention covers all possible combinations of particular and preferred embodiments described herein.

### EXAMPLES

### Example 1. Wet air oxidation

An aqueous stream with the following composition (wt %) and properties was undergone to wet air oxidation: 1,2-propanediol 5.0%, sodium phenolate 2.0%, sodium benzoate 3.0%, sodium acetate 1.1%, pH 13 and Chemical Oxygen Demand (COD) 18.2%. The wet air oxidation was carried out at 95 barg, 290 °C and a residence time of 85 min.

The COD reduction was about 70%. The effluent thus obtained (1), once depressurized and cooled had the following composition (wt %) and properties: sodium acetate 2.7%, sodium benzoate 1.1 %, sodium propionate <0.1 %, sodium formiate <0.1 %, sodium carbonate 0.8%, pH 8 and COD 4.9%.

### Example 2. Benzoic acid precipitation

The effluent (1) obtained in example 1 was cooled at 10°C (A). Sulphuric acid 98% wt was added (2), 41.7 g/kg of effluent, and mixed during 60 min (B). Thus, 7.8 g of CO₂ / kg effluent was obtained as a consequence of the acidification of carbonate until pH 5 (3); a suspension was obtained by precipitation of 6.9 g acid/ L effluent from pH 4.5, and the stream was finally acidified until pH 2. The precipitated acid had a salt content of 19% wt with 63000 ppm of sodium and 1000 ppm molybdenum in dry basis. The benzoic acid thus precipitated was undergone to a filtration step through a polymeric membrane with 0.45 µm average pore size (C).

### Example 3. Benzoic acid precipitation

The effluent (1) obtained in example 1 was precipitated at room temperature. Sulphuric acid 98% wt was added (2), 41.7 g/kg of effluent, and mixed during 60 min (B). Thus, 7.8 g of CO₂ / kg effluent was obtained as a consequence of the acidification of carbonate until pH 5 (3); a suspension was obtained by precipitation of 5.6 g / L effluent from pH 4.5, and the stream was finally acidified until pH 2. The precipitated acid had a salt content of 9% wt with 28000 ppm of sodium and 600 ppm molybdenum in dry basis. The benzoic acid thus precipitated was undergone to a filtration step through a polymeric membrane with 0.45 µm average pore size (C).

### Example 4. Benzoic acid purification

Benzoic acid obtained in example 2 was purified at room temperature by resuspension and extraction with demineralised water (4) in a water : solid mass ratio of 8.5. The aqueous suspension obtained was undergone to mixing during 10 min and filtration (D). 5.5 g benzoic acid by kg effluent was obtained. Pure benzoic acid (5) thus obtained had the following composition in dry basis (wt %): benzoic acid 98%, sodium <3000 ppm, molybdenum <100 ppm.

### Example 5. Benzoic acid purification

Benzoic acid obtained in example 2 was purified at 10 °C by extractive filtering with demineralised water (4) in a water : solid mass ratio of 16. The water passes through a filter cake (D). 5.9 g benzoic acid by kg effluent was obtained. Pure benzoic acid (5) thus obtained had the following composition in dry basis (wt %): benzoic acid 98%, sodium <1000 ppm, molybdenum <20 ppm.

### Example 6. Molybdenum recovery

The filtrate obtained in example 2, was undergone to ionic exchange in order to retain the molybdenum values. The ionic exchange equipment used consist of a carrousel with 3 ionic exchange beds (E, F, G) packed with a macroporous weak anionic exchange resin, Purolite® PFA-133Mo (The Purolite Company, USA). The filtrate was passed through 2 beds (F, G) during 8 hours each at a volumetric speed of 5 BV/h and descending flow. Molybdenum concentration was reduced from 350 ppm to less than 30 ppm in the first bed (F) and less than 2 ppm in the second (G), to obtain a stream with reduced molybdenum content (6).

### Example 7. Molybdenum recovery

Precipitation of benzoic acid as in example 2 was repeated but increasing the added acid amount until pH 1. The filtrate thus obtained was undergone to ionic exchange in order to retain the molybdenum values. The ionic exchange equipment used consist of a carrousel with 3 ionic exchange beds (E, F, G) packed with a macroporous weak anionic exchange resin, Purolite® A-100 (The Purolite Company, USA). The filtrate was passed through 2 beds (F, G) during 8 hours each at a volumetric speed of 3 BV/h and descending flow. Molybdenum concentration was reduced from 350 ppm to less than 30 ppm in the first step (F) and less than 2 ppm in the second (G), to obtain a stream with reduced molybdenum content (6).

### Example 8. Molybdenum recovery

Precipitation of benzoic acid as in example 2 was repeated but reducing the added acid amount until pH 3. The filtrate thus obtained was undergone to ionic exchange in order to retain the molybdenum values. The ionic exchange equipment used consist of a carrousel with 3 ionic exchange beds (E, F, G) packed with a macroporous weak anionic exchange resin, Purolite® A-170 (The Purolite Company, USA). The filtrate was passed through one bed (F) during 8 hours at a volumetric speed of 3 BV/h and descending flow. Molybdenum concentration was reduced from 350 ppm to less than 30 ppm (6).

### Example 9. Molybdenum concentration

The effluent obtained in example 6 was fractionated as a function of its molybdenum concentration. The most concentrated fraction, with at least 5000 ppm molybdenum, was obtained between 15-45 min. of regeneration. The fraction obtained was undergone to a molybdenum concentration process in various steps. In the first step (H) the concentrate was undergone to a chemical precipitation adding ferric sulphate 45% wt solution (8), 8 mol iron by mol of molybdenum to recover. The pH is adjusted with sulphuric acid 98% wt or sodium hydroxide 20% wt at 3.5. After 10 min at room temperature, 93% molybdenum was precipitated as iron-molybdenum oxide.

### Example 10. Molybdenum concentration

The effluent obtained in example 6 was fractionated as a function of its molybdenum concentration. The most concentrated fraction, with at least 5000 ppm molybdenum, was obtained between 15-45 min. of regeneration.

The fraction obtained was undergone to a molybdenum concentration process in various steps. In the first step (H) the concentrate was undergone to a chemical precipitation adding ferric sulphate 45% wt solution (8), 6 mol iron by mol of molybdenum to recover. The pH is adjusted with sulphuric acid 98% wt or sodium hydroxide 20% wt at 3.2. After 10 min at 80 °C, 80% molybdenum was precipitated as iron-molybdenum oxide.

### Example 11. Molybdenum concentration

The effluent obtained in example 6 was fractionated as a function of its molybdenum concentration. The most concentrated fraction, with at least 5000 ppm molybdenum, was obtained between 15-45 min. of regeneration.

The fraction obtained was undergone to a molybdenum concentration process in various steps. In the first step (H) the concentrate was undergone to a chemical precipitation adding calcium chloride (8), 15 mol of calcium by mol of molybdenum to recover. The pH is adjusted with sodium hydroxide 20% wt at 12. After 10 min at room temperature (25 °C), 95% molybdenum was precipitated as calcium molybdate.

### Example 12. Molybdenum concentration

The suspension obtained in example 9 was undergone to a second precipitation step (I) in order to reduce the iron in excess from the first precipitation step. Calcium hydroxide 20% wt was added until pH 7 (9). After 10 min at room temperature (25 °C) the iron concentration was lowered in liquid phase from 2500 ppm to <50 ppm. The yield of molybdenum recovery was increased 2%.

### Example 13. Molybdenum concentration

The suspension obtained in example 12 was undergone to a solid-liquid separation step (J), centrifugal separation at 2770g during 10 min. A molybdenum concentrated phase with at least 20% solids and a clear phase (10) were obtained. The concentrated phase was undergone to a drying step (K) to concentrate the solids to 80% (c12). The concentrated phase thus obtained had a molybdenum content of 7% in dry basis (13).

### Example 14. Molybdenum concentration.

The effluent obtained in example 6 was fractionated as a function of its molybdenum concentration. The most concentrated fraction, with at least 5000 ppm molybdenum, was obtained between 15-45 min. of regeneration.

The fraction obtained (11) was undergone to a concentration step by evaporation at 105 °C. After elimination of water (12), a concentrated phase was obtained with at least 11 % molybdenum (13).

## Claims

1. Process for recovering molybdenum and benzoic acid from the alkaline aqueous stream from a epoxidation process, which comprises the following steps:
(i) wet air oxidation of the alkaline aqueous stream;
(ii) acidifiyng the stream generated in step (i) with an acid at a pH comprised of from 1.0 to 4.5 obtaining a solid-phase fraction containing impure benzoic acid and a liquid-phase fraction containing molybdenum;
(iii) separating the solid-phase fraction from the liquid-phase fraction of the step (ii);
(iv) washing the solid-phase fraction separated in step (iii) with demineralised water to recover the benzoic acid;
(v) passing the liquid-phase fraction separated in step (iii) through at least one anionic exchange resin to produce a liquid stream with low molybdenum concentration;
(vi) regenerating the anionic exchange resin with a base to recover the molybdenum as molybdate.

2. The process according to claim 1, wherein the wet air oxidation step (i) is performed at a temperature comprised of from 250 to 315°C, a pressure comprised of from 40 to 120 bar, a chemical oxygen demand inlet concentration comprised of from 5 to 17% wt, a reaction time comprised of from 20 to 120 min, and excess of compressed oxygen, air, or their mixture as the oxidant, in order to obtain an oxygen concentration comprised of from 1 to 15% vol in the wet air oxidation off-gas.

3. The process according to any of claims 1-2, wherein the acidification of step (ii) is performed with an acid selected from sulphuric acid, hydrochloric acid, nitric acid, phosphoric acid.

4. The process according to claim 3, wherein the acid is sulphuric acid.

5. The process according to any of claims 1-4, wherein previously to the acidification step (ii) the wet air oxidation effluent of step (i) is cooled down at a temperature comprised of from 2 to 50°C.

6. The process according to any of claims 1-5, wherein the wet air oxidation effluent of step (i) is cooled down at a temperature comprised of from 6 to 15°C and then the stream is acidified at a pH comprised of from 2.0 to 2.5.

7. The process according to any of claims 1-6, wherein the separation step (iii) is performed by filtration with a 0.15 to 100.00 µm average pore size polymeric membrane.

8. The process according to any of claims 1-7, wherein washing step (iv) is performed with demineralised water at a water:solid phase ratio comprised of from 50:1 to 2:1 w/w.

9. The process according to claim 8, which further comprises drying the washed solid phase obtained in step (iv) at a temperature comprised of from 40°C to 80°C and recovering the benzoic acid.

10. The process according to any of claims 1-9, wherein the anionic exchange resin is a weak anionic resin having tertiary, quaternary or complex amine functional groups.

11. The process according to any of claims 1-10, wherein the liquid phase containing molybdenum obtained in step (iii) is treated in a carousel of at least three anionic exchange resin beds.

12. The process according to any of claims 1-11, wherein the recovery of the molybdenum from the anionic exchange resin is performed with a reactant selected from CaCl₂, CaSO₄, Ca(OH)₂, Fe(III) citrate, FeCl₃, Fe₂(SO₄)₃, FeSO₄.

13. The process according to claim 12, wherein the reactant is Fe₂(SO₄)₃.

14. The process according to claim 13, wherein the resulting slurry containing the molybdate compound is contacted with a 20 % Ca(OH)₂ whitewash, and the molybdate containing slurry thus obtained is dried to recover a molybdenum concentrate with a solid content of at least 80 % and a molybdenum content comprised of from 7 to 10 % on dry basis.

15. The process according to any of claims 1-14, further comprising the dissolution in H₂O or in aqueous NaOH of the CO₂ formed during the acidification step (ii).

## Patentansprüche

1. Verfahren zur Rückgewinnung von Molybdän und Benzoesäure aus der alkalischen wässrigen Strömung, die bei einem Epoxidationsverfahren erzeugt wird, welches die folgenden Schritte umfasst:
i) die Nassluftoxidation der alkalischen wässrigen Strömung;
ii) die Säuerung der im Schritt (i) erzeugten Strömung mit einer Säure bei einem pH-Wert, der von 1,0 bis 4,5 reicht, wodurch eine Festphasenfraktion enthaltend verunreinigte Benzoesäure und eine Flüssigphasenfraktion enthaltend Molybdän erhalten werden;
iii) die Abtrennung der Festphasenfraktion aus der Flüssigphasenfraktion des Schritts (ii);
iv) das Waschen der im Schritt (iii) abgetrennten Festphasenfraktion mit demineralisiertem Wasser, um die Benzoesäure zurückzugewinnen;
v) der Durchgang der im Schritt (iii) abgetrennten Flüssigphasenfraktion durch mindestens ein Anionenaustauschharz, um eine flüssige Strömung mit einer geringen Konzentration an Molybdän zu gewinnen;
vi) die Regeneration des Anionenaustauschharzes mit einem Alkali, um das Molybdän als Molybdat zurückzugewinnen.

2. Das Verfahren nach Anspruch 1, wobei der Nassluftoxidationsschritt (i) bei einer Temperatur reichend von 250 bis 315 °C, bei einem Druck reichend von 40 bis 120 bar, bei einer Chemischen Sauerstoffbedarf (Eintrittskonzentration) reichend von 5 Gew.-% bis 17 Gew.-%, bei einer Reaktionszeit reichend von 20 bis 120 min, und mit einem Überschuss von komprimierten Sauerstoff, Luft oder deren Mischung als Oxidationsmittel durchgeführt wird, um eine Sauerstoffkonzentration reichend von 1 Vol.-% bis 15 Vol.-% im Abgas der Nassluftoxidation zu erhalten.

3. Das Verfahren nach einem der Ansprüche 1-2, wobei die Säuerung des Schritts (ii) mit einer Säure durchgeführt wird, die aus Schwefelsäure, Chlorwasserstoffsäure, Salpetersäure, Phosphorsäure ausgewählt ist.

4. Das Verfahren nach Anspruch 3, wobei die Säure Schwefelsäure ist.

5. Das Verfahren nach einem der Ansprüche 1-4, wobei der Nassluftoxidationsabfluss des Schritts (i) vor dem Säuerungsschritt (ii) bis auf einer Temperatur reichend von 2 bis 50 °C abgekühlt wird.

6. Das Verfahren nach einem der Ansprüche 1-5, wobei der Nassluftoxidationsabfluss des Schritts (i) bis auf einer Temperatur reichend von 6 bis 15 °C abgekühlt wird und die Strömung dann bis auf einen pH-Wert reichend von 2,0 bis 2,5 gesäuert wird.

7. Das Verfahren nach einem der Ansprüche 1-6, wobei der Abtrennungsschritt durch Filtration mit einer Polymermembran mit einem mittleren Porengröße von 0,15 bis 100,00 µm durchgeführt wird.

8. Das Verfahren nach einem der Ansprüche 1-7, wobei der Waschschritt (iv) mit demineralisiertem Wasser bei einem Gewichtsverhältnis vom Wasser zur Festphase von 50:1 bis 2:1 durchgeführt wird.

9. Das Verfahren nach Anspruch 8, welches weiterhin das Trocknen der im Schritt (iv) erhaltenen gewaschenen Festphase bei einer Temperatur reichend von 40 °C bis 80 °C und die Rückgewinnung der Benzoesäure umfasst.

10. Das Verfahren nach einem der Ansprüche 1-9, wobei das Anionenaustauschharz ein schwach anionisches Harz mit tertiären, quaternären oder komplexen Amin-Funktionsgruppe ist.

11. Das Verfahren nach einem der Ansprüche 1-10, wobei die im Schritt (iii) erhaltene flüssige Phase, die Molybdän enthält, in einem Karussell von mindestens drei anionischen Austauschharzbetten behandelt wird.

12. Das Verfahren nach einem der Ansprüche 1-11, wobei die Rückgewinnung des Molybdäns aus dem Anionenaustauschharz mit einem Reagenz durchgeführt wird, das aus CaCl₂, CaSO₄, Ca(OH)₂, Fe(III)-Citrat, FeCl₃, Fe₂(SO₄)₃, FeSO₄ ausgewählt ist.

13. Das Verfahren nach Anspruch 12, wobei das Reagenz Fe₂(SO₄)₃ ist.

14. Das Verfahren nach Anspruch 13, wobei die resultierende Suspension, welche die Molybdatverbindung enthält in Kontakt mit einer 20%igen Ca(OH)₂ Kalkmilch gebracht wird, und die dadurch erhaltene Suspension, welche das Molybdat enthält, getrocknet wird, um ein Molybdän-Konzentrat mit einem Gehalt an Feststoffen von mindestens 80% und einem Molybdängehalt von 7 bis 10% der Trockenmasse zurückzugewinnen.

15. Das Verfahren nach einem der Ansprüche 1-14, weiterhin umfassend die Lösung des im Säuerungsschritt (ii) erzeugten CO₂ in H₂O oder in wässrigem NaOH.

## Revendications

1. Procédé de récupération de molybdène et d'acide benzoïque à partir du flux aqueux alcalin généré dans un procédé d'époxidation, comprend le procédé les étapes suivantes :
i) oxydation à l'air humide du flux aqueux alcalin ;
ii) acidification du flux généré dans l'étape (i) avec un acide à un pH allant de 1,0 à 4,5, obtenant une fraction de phase solide contenant de l'acide benzoïque impure et une fraction de phase liquide contenant du molybdène ;
iii) séparer la fraction de phase solide de la fraction de phase liquide de l'étape (ii) ;
iv) laver la fraction de phase solide séparée dans l'étape (iii) avec de l'eau déminéralisée afin de récupérer l'acide benzoïque ;
v) faire passer la fraction de phase liquide séparée dans l'étape (iii) à travers au moins une résine échangeuse d'anions afin de produire un flux liquide avec molybdène à faible concentration ;
vi) régénérer la résine échangeuse d'anions avec une base afin de récupérer le molybdène en tant que molybdate.

2. Le procédé selon la revendication 1, dans lequel l'étape (i) d'oxydation à l'aire humide est effectuée à une température comprise de 250 à 315 °C, à une pression comprise de 40 à 120 bar, à une concentration d'entrée de la demande chimique en oxygène comprise de 5 à 17% en poids, avec un temps de réaction compris de 20 à 120 min, et avec un excès d'oxygène comprimé, d'aire, ou de leur mélange en tant que l'oxydant, afin d'obtenir une concentration en oxygène comprise de 1 à 15% en volume dans l'effluent gazeux de l'oxydation à l'air humide.

3. Le procédé selon l'une quelconque des revendications 1-2, dans lequel l'acidification de l'étape (ii) est effectuée avec un acide choisi parmi l'acide sulfurique, l'acide chlorhydrique, l'acide nitrique, l'acide phosphorique.

4. Le procédé selon la revendication 3, dans laquelle l'acide est l'acide sulfurique.

5. Le procédé selon l'une quelconque des revendications 1-4, dans lequel avant de l'étape (ii) d'acidification l'effluent de l'oxydation à l'air humide de l'étape (i) est refroidi à une température comprise de 2 à 50 °C.

6. Le procédé selon l'une quelconque des revendications 1-5, dans lequel l'effluent de l'oxydation à l'air humide de l'étape (i) est refroidi à une température comprise de 6 à 15 °C et puis le flux est acidifié à un pH comprise de 2,0 à 2,5.

7. Le procédé selon l'une quelconque des revendications 1-6, dans lequel l'étape de séparation est effectuée par filtration avec une membrane polymère avec une taille moyenne de pores allant de 0,15 à 100,00 µm.

8. Le procédé selon l'une quelconque des revendications 1-7, dans lequel l'étape (iv) de lavage est effectuée avec de l'eau déminéralisée avec un rapport eau:phase solide allant de 50:1 à 2:1 en poids.

9. Le procédé selon la revendication 8, qui comprend en outre sécher la phase solide lavée obtenue dans l'étape (iv) à une température comprise de 40 °C à 80 °C et récupérer l'acide benzoïque.

10. Le procédé selon l'une quelconque des revendications 1-9, dans lequel la résine échangeuse d'anions est une résine anionique faible ayant des groupes fonctionnels amine tertiaires, quaternaires ou complexes.

11. Le procédé selon l'une quelconque des revendications 1-10, dans lequel la phase liquide contenant le molybdène obtenue dans l'étape (iii) est traitée dans un carrousel d'au moins trois lits de résines échangeuses d'anions.

12. Le procédé selon l'une quelconque des revendications 1-11, dans lequel la récupération du molybdène à partir de la résine échangeuse d'anions est effectuée avec un réactif choisi parmi le CaCl₂, le CaSO₄, le Ca(OH)₂, le citrate de Fe(III), le FeCl₃, le Fe₂(SO₄)₃, le FeSO₄.

13. Le procédé selon la revendication 12, dans laquelle le réactif est le Fe₂(SO₄)₃.

14. Le procédé selon la revendications 13, dans lequel la suspension résultante contenant le composé de molybdate est mise en contact avec une eau de chaux à 20% en Ca(OH)₂, et la suspension contenant le molybdate ainsi obtenue est séchée afin de récupérer un concentré de molybdène avec une teneur en matières solides d'au moins 80% et une teneur en molybdène allant de 7 à 10% sur une base sèche.

15. Le procédé selon l'une quelconque des revendications 1-14, comprenant en outre la dissolution du CO₂ formé pendant l'étape (ii) d'acidification dans H₂O ou dans NaOH aqueux.
